# FASCICULE DE BREVET EUROPEEN

(11) **EP 1 549 252 B1**
(45) Date de publication et mention de la délivrance du brevet: **09.01.2008**
(21) Numéro de dépôt: 03780211.3
(22) Date de dépôt: 10.10.2003
(51) Int. Cl.: A61F 2/06, A61B 17/11

(54) **DISPOSITIF DE LIAISON ENTRE UN CONDUIT CORPOREL ET UNE PROTHESE**
VERBINDUNGSVORRICHTUNG ZWISCHEN EINEM KÖRPERKANAL UND EINER PROTHESE
DEVICE FOR CONNECTION BETWEEN A CORPOREAL DUCT AND A PROSTHESIS

(30) Priorité: 10.10.2002 FR 0212601
(43) Date de publication de la demande: 06.07.2005
(73) Titulaire: Garbe, Jean-François, 47000 Agen (FR)
(72) Inventeur: Garbe, Jean-François, 47000 Agen (FR)
(74) Mandataire: Schmit, Christian Norbert Marie
(86) Numéro de dépôt international: PCT/FR2003/002988
(87) Numéro de publication internationale: WO 2004/032800

(56) Documents cités:
- WO-A-98/19631
- US-A- 5 931 842
- US-A1- 2001 044 637
- US-A1- 2002 087 176

## Description

La présente invention se rapporte à la mise en place d'une prothèse tubulaire rapportée soit à une extrémité d'un conduit corporel, soit entre deux conduits corporels à relier, par intubation de l'une des extrémités, ou des deux, de la prothèse dans le ou les conduits corporels et fixation des parties intubées à l'aide d'un dispositif de liaison objet de l'invention (par exemple voir US-A-5931842).

Dans le domaine des anastomoses entre conduits corporels, une première solution consiste à relier directement les conduits entre eux à l'aide de sutures manuelles chirurgicales.

Cette solution a pour seul avantage de ne nécessiter aucun appareillage. Toutefois, le temps opératoire pour la réaliser est relativement long et la qualité de la jonction dépend de la dextérité du praticien qui doit relier deux conduits souples entre eux.

Une autre solution consiste à utiliser une prothèse tubulaire, sous forme d'un manchon dont les deux extrémités sont intubées respectivement dans les deux conduits à relier, des moyens étant prévus pour solidariser entre elles les parties intubées de la prothèse et les parties terminales desdits conduits.

Par le document WO 98/19631 on connaît un dispositif d'anastomose du type ci-dessus constitué d'une prothèse formée d'une structure maillée expansible dont les extrémités intubées dans les conduits à relier sont pourvues de moyens d'ancrage dans les parties terminales desdits conduits.

A cet effet, chaque extrémité de la prothèse est constituée d'une structure spéciale auto-expansible munie de saillies radiales destinées à pénétrer dans le tissu des conduits pour éviter tout glissement entre ces derniers et la prothèse.

Un tel dispositif n'est pas pleinement satisfaisant. D'une part, parce qu'il nécessite la réalisation d'une prothèse spéciale puisqu'elle intègre à chaque extrémité des structures particulières. D'autre part, parce-que la mise en place de la prothèse ne peut pas être effectuée à l'aide d'un cathéter à ballonnet, les parties intubées de la prothèse étant plaquées contre les parties terminales des conduits par simple auto-expansion, cette technique n'assurant pas un ancrage véritablement ferme et étanche.

Par ailleurs, on connaît également par le document US 4 214 587 un dispositif d'anastomose de deux vaisseaux à l'aide d'un élément annulaire compressible élastiquement radialement pourvu extérieurement de picots. Cet élément doit être comprimé pour réduire son diamètre afin d'être introduit dans la partie terminale du conduit à anastomoser. Une fois relâché, il s'expanse pour prendre son diamètre nominal.

Outre, l'inconvénient d'un ancrage non assuré d'être véritablement ferme et étanche comme dans le cas précédent, l'élément annulaire cessant de s'expanser alors que la partie intubée n'est peut-être pas parfaitement plaquée contre la partie terminale du conduit à relier, un tel élément est dimensionné nécessairement pour une plage très réduite de diamètres de conduits à anastomoser, ce qui impose de réaliser une gamme d'éléments annulaires de divers diamètres et de choisir avec précision l'élément le plus approprié à chaque anastomose.

Enfin, par les documents US 5 931 842 et WO 98/19634, on connaît des systèmes destinés à réaliser des anastomoses mais dans le cadre particulier de pontages cardiaques, c'est-à-dire d'anastomose termino-latérale, à l'aide d'anneaux expansibles pourvus extérieurement de picots, dont aucun détail n'est donné quant à la structure de l'anneau et l'agencement des picots.

La présente invention vise à pallier les inconvénients des systèmes d'anastomose connus et plus particulièrement à proposer un dispositif spécifiquement adapté aux anastomoses termino-terminales.

A cet effet, l'invention a pour objet un dispositif de liaison entre les extrémités préalablement intubées d'un conduit corporel et d'une prothèse de forme sensiblement tubulaire, ce dispositif étant constitué d'un manchon à structure maillée ou analogue déformable à l'aide d'un cathéter à ballonnet et susceptible d'une expansion radiale entre une configuration stable de diamètre minimal et une configuration finale après expansion également stable, ledit manchon comportant, à chaque extrémité, une série de picots de transfixion des parties en recouvrement du manchon, alignés régulièrement en couronne et orientés radialement, caractérisé en ce que lesdits picots présentant un profil hémostatique comprenant une partie de base de section circulaire prolongée par une partie terminale de forme triédrique.

Suivant un mode de réalisation préféré, le manchon expansible est constitué d'un cylindre d'acier ajouré suivant des découpes losangées et les picots sont rapportés et fixés par soudage ou collage aux intersections des côtés desdits losanges.

Suivant une autre caractéristique du dispositif de l'invention, ledit manchon est susceptible de s'expanser dans un rapport diamètre en situation de transfixion sur diamètre initial supérieur à 2.

Suivant un mode de réalisation- préférentiel la partie hors-extrémités du manchon est également pourvue de picots de transfixion.

De préférence, les picots en couronne des extrémités du manchon sont rectilignes et les autres picots sont légèrement incurvés avec leur pointe orientée vers l'une ou l'autre des extrémités du manchon ou toute autre direction, de manière aléatoire.

L'invention s'applique à la liaison entre une extrémité d'une prothèse et la partie terminale d'un conduit corporel tel qu'une artère de diamètre entre 6 mm et 30 mm, mais elle s'applique également à la jonction entre deux conduits corporels via une prothèse dont les deux extrémités sont intubées dans les parties terminales des deux conduits à abouter.

D'autres caractéristiques et avantages ressortiront de la description qui va suivre d'un mode de mise en oeuvre de l'invention, description donnée à titre d'exemple uniquement, en regard des dessins annexés sur lesquels:
- la figure 1 A est une vue en perspective d'un mode de réalisation du dispositif de liaison de l'invention dans sa configuration de diamètre minimal,
- la figure 1B représente le dispositif de la figure 1 A déployé dans sa configuration de diamètre maximal,
- la figure 2A est une vue en élévation d'un picot,
- la figure 2B illustre le profil triédrique de la pointe du picot de la figure 2A,
- la figure 3 est une vue illustrant la fixation d'une prothèse sur un conduit corporel à l'aide du dispositif de liaison de l'invention,
- la figure 4 est un schéma illustrant la mise en place et la fixation d'un premier conduit corporel à une première extrémité d'une prothèse,
- la figure 5 est un schéma illustrant la mise en place et la fixation d'un second conduit corporel à la seconde extrémité de la prothèse, et
- la figure 6 illustre l'application du procédé à la mise en place de dispositifs selon l'invention sur une prothèse bifurquée.

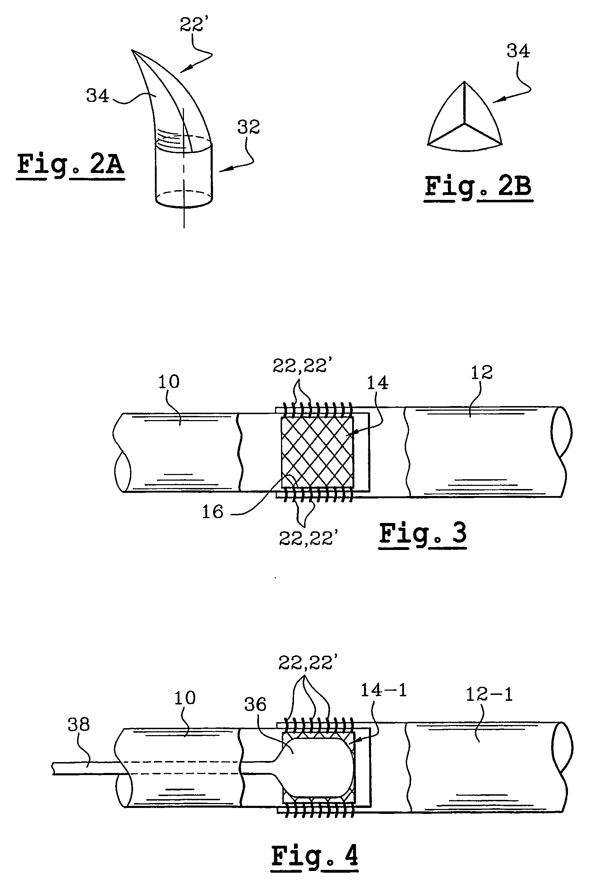

Comme illustré sur la figure 3, le dispositif de l'invention permet d'assurer la liaison entre une prothèse 10 et un conduit corporel 12, dans lequel l'une des extrémités de la prothèse 10 est intubée.

Ce dispositif de liaison- plus particulièrement destiné aux conduits vasculaires peut s'adapter à tout autre conduit corporel dans lequel est susceptible d'être intubée une prothèse.

La prothèse de forme adaptée au conduit corporel, généralement sensiblement tubulaire, n'est pas décrite plus en détail car à la portée de l'homme de l'art. Selon un mode de réalisation connu, cette prothèse est le plus souvent en DACRON^{®} . Il peut s'agir d'une prothèse tubulaire droite ou bifurquée en Y.

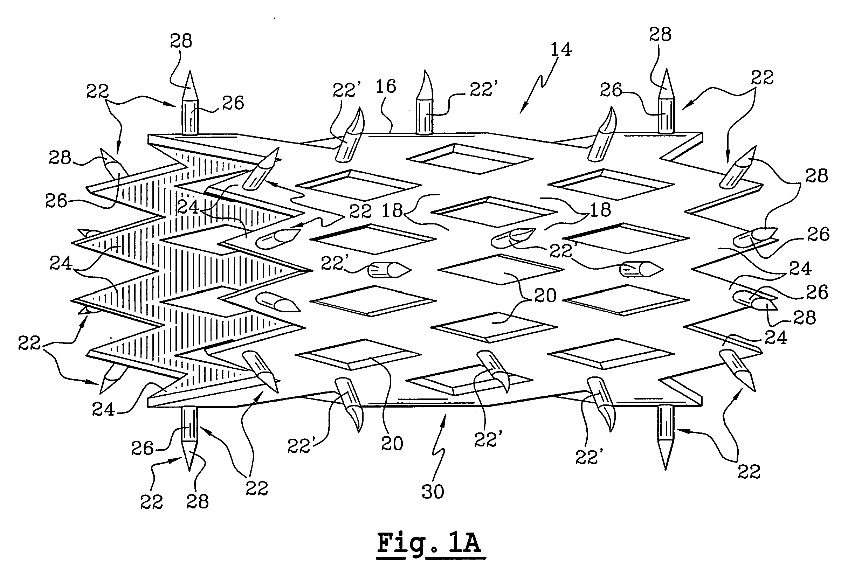

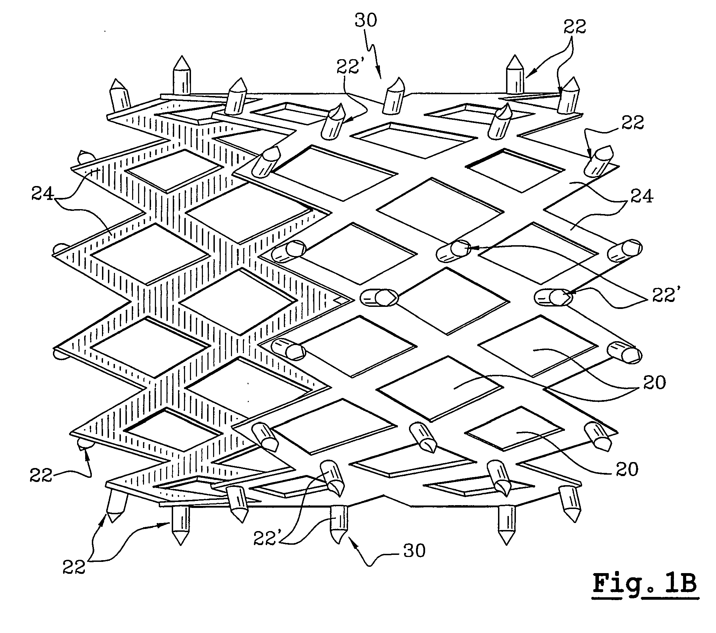

Selon l'invention, le dispositif de liaison 14 entre la prothèse 10 et le conduit corporel 12 comprend un manchon expansible 16 disposé à l'intérieur de la prothèse dont un mode de réalisation est représenté sur les figures 1 A et 1B.

Sur la figure 1 A est représenté schématiquement un manchon tubulaire 1 6 en acier ajouré dans sa configuration de diamètre minimal, c'est-à-dire tel qu'il se présente en fin de fabrication, prêt à être utilisé.

Le manchon 16 est par exemple réalisé par découpe au laser d'un tube d'acier approprié d'épaisseur de paroi de quelques dixièmes de millimètre, de diamètre 8 mm et de longueur 24 mm. Les découpes sont losangées en sorte de réaliser une structure maillée régulière dont les branches 18 séparant les losanges 20 présentent, à titre illustratif, une longueur de quelques millimètres par exemple 4 mm pour une largeur de l'ordre de quelques dixièmes de millimètre. Il est à noter que les figures 1 A et 1 B illustrent simplement la structure générale du manchon 16 et que les rapports dimensionnels indiqués ci-dessus ne sont pas respectés sur les dessins.

Conformément à l'invention, aux deux extrémités du manchon 1 6 des picots 22 de transfixion sont régulièrement répartis en couronne radialement vers l'extérieur à partir de la face externe du manchon.

Les picots 22 sont en acier et rapportés par exemple par soudage ou collage sur les pointes d'extrémité 24 du manchon 16.

Les picots 22 ont une longueur comprise entre environ 0,5 mm et 3 mm et sont rectilignes. Leur profil est hémostatique et comprend une partie de base 26 cylindrique, d'un diamètre de l'ordre de quelques dixièmes de millimètre, prolongée par une partie terminale 28 de forme triédrique. De même que la structure maillée les picots 22 des figures 1 A et 1 B ne sont pas représentés en vraie grandeur.

De préférence, toutes les pointes 24 des deux extrémités du manchon 16 sont munies de picots 22.

Les picots 22 des extrémités peuvent être de hauteur réduite par rapport à celle des picots de la zone intermédiaire. En effet, lorsque la longueur du manchon 16 est supérieure à la longueur des parties intubées en recouvrement, les parties d'extrémité du manchon se trouvent en regard d'une seule paroi à transfixier en sorte que les picots dans ces zones peuvent avoir une hauteur réduite, ayant moins d'épaisseur de paroi à traverser que les autres picots du manchon.

Sur la face externe du manchon 16 délimitée entre les deux couronnes de picots 22 d'extrémité sont également, dans le mode de réalisation représenté, implantés des picots 22', aux intersections des branches 18 de la structure maillée et plus précisément à certaines intersections seulement.

Dans la zone centrale du manchon 16 est agencée une couronne circulaire 30 de picots 22', à raison d'un picot toutes les deux intersections. Entre la couronne centrale 30 et chaque couronne de picots 22 d'extrémité est agencée une autre couronne de picots 22' identique à la couronne 30.

La distribution des picots 22' est régulière ou non.

De préférence, les picots 22' ont le profil représenté en figure 2A et 2B, à savoir un profil hémostatique du type des picots 22, à base 32 cylindrique prolongée par une pointe terminale à profil triédrique 34 (figure 2B).

En outre, la pointe terminale 34 est de préférence incurvée en direction de l'une ou l'autre des extrémités du manchon 16 ou selon toute autre direction, les picots 22' ayant de préférence des orientations différentes, l'inclinaison desdites parties terminales 34 étant comprise entre 0 et 10° et, de préférence de l'ordre de 5°.

La figure 1 B représente le manchon 16 de la figure 1 A à l'état expansé, la longueur de la structure maillée étant réduite de 24 mm à 20 mm et le diamètre passant de 8 mm à 20 mm.

Ladite structure n'est pas élastique, ne présente pas de mémoire de forme et est stable dimensionnellement quelle que soit l'amplitude de l'expansion qui lui sera donnée, cette dernière lui étant appliquée à l'aide d'un ballon gonflable d'un cathéter à ballonnement conventionnel, lors de la mise en place du dispositif de liaison de l'invention comme on va maintenant le décrire en référence aux figures 3 à 5.

L'utilisation d'un tel dispositif de liaison est relativement simple et décrite au regard de la figure 3. De manière connue, dans le conduit corporel 12 est intubée l'extrémité de la prothèse 10, sur une longueur de l'ordre de 25 mm. Le dispositif de liaison 14, très schématisé sur la figure 3, est disposé à l'intérieur de la prothèse 10 au droit de la zone de recouvrement du conduit corporel 12 et de la prothèse 10. Lors de l'expansion du manchon 16, les picots 22, 22' perforent à la fois la prothèse et le conduit corporel de manière à assurer la liaison entre les deux éléments.

L'invention s'applique également à l'anastomose de deux conduits corporels via une prothèse 10 et 12.2 dont les deux extrémités sont intubées dans les parties terminales desdits conduits.

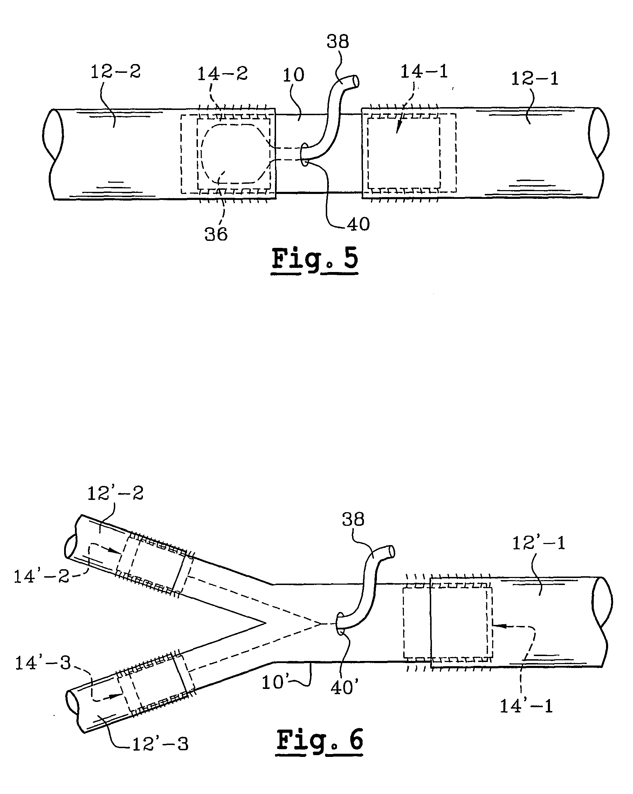

Comme illustré par les figures 4 et 5, le dispositif de connexion comprend deux dispositifs de liaison 14-1 et 14-2 constitués par exemple par un manchon 16 du type de la figure 1, disposés respectivement au niveau de chaque extrémité à l'intérieur de la prothèse 10.

L'invention a également pour objet un procédé de mise en place des dispositifs de liaison.

A cet effet, dans le conduit corporel 12.1 est intubée une première extrémité de la prothèse 10. Le premier dispositif de liaison 14.1 est introduit à l'intérieur de la prothèse 10 par la seconde extrémité, et mis en place, comme illustré par la figure 4 à l'aide d'un ballon gonflable 36 rapporté sur le cathéter 38 de mise en place à la manière connue.

Puis conformément à l'invention, dans le second conduit corporel 12.2 est ensuite intubée la seconde extrémité de la prothèse 10, puis le second dispositif de liaison 14.2 (figure 5) est introduit via un orifice 40 ménagé au niveau de la prothèse 10, refermé après la mise en place, par des points de suture manuelle.

Grâce à l'utilisation du dispositif de liaison de l'invention, les temps opératoires nécessaires sont réduits, permettant de réduire les risques de mortalité.

Sur la figure 6, on a représenté une prothèse bifurquée en Y 10' dont une première extrémité est intubée dans un premier conduit corporel 12'-1 et fixée à l'aide d'un premier manchon 14'-1 selon l'invention à l'aide du cathéter 38 introduit dans la prothèse 10' par l'une des extrémités bifurquées. Les deux extrémités bifurquées de la prothèse 10' sont intubées dans deux autres conduits corporels 12'-2 et 12'-3 et fixées à l'aide de deux autres manchons 14'-2 et 14'-3 qui sont mis en place successivement comme dans le cas illustré par la figure 5 par introduction du cathéter 38 muni du ballon 36 sur lequel est enfilé le manchon de transfixion (14'-2, 14'-3), au travers d'un orifice 40' qui sera ensuite refermé.

Du fait que le manchon 16 peut s'expanser dans une plage de diamètres importante, le rapport entre le diamètre final, in situ, du manchon et le diamètre initial étant avantageusement supérieur à 2, et du fait que l'état final est stable puisqu'il n'y a pas de rétraction du manchon une fois le ballon de mise en place dégonflé, on réalise un plaquage efficace du manchon 1 6 contre les parties intubées en regard, à la fois étanche et ferme grâce aux picots 22, 22' de transfixion desdites parties intubées.

De plus, l'aptitude du manchon 16 à une expansion d'amplitude variable permet à l'aide d'un manchon de taille unique d'être utilisé pour des anastomoses par exemple de vaisseaux dont les diamètres peuvent varier dans une plage étendue, par exemple des artères de diamètre compris entre 6 et 30 mm.

On peut cependant et bien entendu, suivant les applications, réaliser des manchons 16 de différentes tailles et comportant des picots 22, 22' de différentes dimensions et distribués sur le manchon de différentes manières.

## Revendications

1. Dispositif de liaison entre les extrémités préalablement intubées d'un conduit corporel (12) et d'une prothèse (10) de forme sensiblement tubulaire, ce dispositif étant constitué d'un manchon (16) à structure maillée ou analogue déformable à l'aide d'un cathéter à ballonnet et susceptible d'une expansion radiale entre une configuration stable de diamètre minimal et une configuration finale après expansion également stable, ledit manchon (16) comportant, à chaque extrémité, une série de picots (22) de transfixion des parties en recouvrement du manchon, alignés régulièrement en couronne et orientés radialement, **caractérisé en ce que** que lesdits picots (22) présentant un profil hémostatique comprenant une partie de base (32) de section circulaire prolongée par une partie terminale (34) de forme triédrique.

2. Dispositif suivant la revendication 1, **caractérisé en ce que** le manchon expansible (16) est constitué d'un cylindre d'acier ajouré suivant des découpes losangées (20) et les picots (22), sont rapportés et fixés par soudage ou collage aux intersections des côtés desdits losanges (20).

3. Dispositif suivant l'une des revendications 1 ou 2 **caractérisé en ce que** ledit manchon (16) est susceptible de s'expanser dans un rapport diamètre en situation de transfixion sur diamètre initial supérieur à 2.

4. Dispositif suivant la revendication 1 à 3, **caractérisé en ce que** la partie hors-extrémités du manchon (16) est également pourvue de picots de transfixion (22').

5. Dispositif suivant la revendication 4, **caractérisé en ce que** les picots (22) en couronne des extrémités du manchon (16) sont rectilignes et les autres picots de transfixion (22') placés sur la partie hors-extrémités du manchon (16) sont légèrement incurvés avec leur pointe (34) orientée vers l'une ou l'autre des extrémités du manchon (16) ou toute autre direction, de manière aléatoire.

6. Dispositif suivant la revendication 5, **caractérisé en ce que** lesdits picots de transfixion (22') placés sur la partie hors-extrémités du manchon (16) présentent une partie terminale (34) inclinée d'un angle compris entre 0 et 10°, de préférence 5°.

7. Dispositif suivant l'une des revendications 4 à 6, **caractérisé en ce que** les picots (22) des extrémités du manchon (16) sont de hauteur réduite par rapport à celle des autres picots de transfixion (22') placés sur la partie hors-extrémités du manchon (16).

8. Dispositif de liaison applicable à l'anastomose termino-terminale d'au moins deux conduits corporels (12-1, 12-2 ; 12'-1, 12'-2, 12'-3) par l'intermédiaire d'une prothèse (10,10') dont les extrémités sont intubable dans les parties terminales desdits conduits, **caractérisé en ce qu'**il est constitué d'un manchon (16) selon l'une quelconque des revendications 1 à 7, engageable dans chaque extrémité de la prothèse (10,10') en regard des parties intubées.

## Claims

1. Connection device for connecting between the previously intubated ends of a body duct (12) and of a prosthesis (10) of substantially tubular shape, this device comprising a sleeve (16) with a mesh structure or the like which can be deformed by means of a balloon catheter and is capable of radial expansion between a stable configuration of minimum diameter and a final configuration after expansion which is also stable, said sleeve (16) comprising, at each end, a series of transfixion pins (22) for trans-fixing the parts overlapping the sleeve, said transfixion pins being aligned regularly in a ring shape and being oriented radially, **characterised in that** said transfixion pins (22) have a hemostatic profile comprising a base part (32) of circular cross section which is extended by an end part (34) of trihedral shape.

2. Device according to claim 1, **characterised in that** the expandable sleeve (16) comprises a steel cylinder perforated with diamond-shaped cutouts (20) and the transfixion pins (22) are attached and fixed by welding or gluing at the intersections of the sides of said diamond-shaped cutouts (20).

3. Device according to one of claims 1 or 2, **characterised in that** said sleeve (16) is capable of expanding in a ratio of the diameter in the fixing situation to the initial diameter of more than 2.

4. Device according to claims 1 to 3, **characterised in that** the intermediate part of the sleeve (16) is also provided with fixing transfixion pins (22').

5. Device according to claim 4, **characterised in that** the transfixion pins (22) in a ring on each end of the sleeve (16) are straight and the other transfixion pins (22') placed on the intermediate part of the sleeve (16) are slightly curved with their point (34) oriented towards one or another end of the sleeve (16) or randomly in any other direction.

6. Device according to claim 5, **characterised in that** said transfixion pins (22') placed on the intermediate part of the sleeve (16) have an end part (34) which is inclined at an angle between 0 and 10°, preferably 5°.

7. Device according to one of claims 4 to 6, **characterised in that** the transfixion pins (22) at the ends of the sleeve (16) are of reduced height compared to that of the other transfixion pins (22') placed on the intermediate part of the sleeve (16).

8. Connection device which can be used for end-to-end anastomosis of at least two body ducts (12-1, 12-2; 12'-1, 12'-2, 12'-3) via a prosthesis (10, 10'), the ends of which can be intubated in the end parts of said ducts, **characterised in that** said device comprises a sleeve (16) according to any one of claims 1. to 7 which can be engaged in each end of the prosthesis (10, 10') facing the intubated parts.

## Patentansprüche

1. Verbindungsvorrichtung zwischen den zuvor intubierten Endstücken eines Körperkanals (12) und einer deutlich rohrförmigen Prothese (10), bestehend aus einer Muffe (16) aus maschenförmiger oder entsprechender Oberflächenstruktur, welche mittels eines Ballonkatheters durch radiale Erweiterung von einer stabilen Konstellation mit minimalem Durchmesser bis zu einer finalen, ebenfalls stabilen Konstellation nach Erweiterung verformt werden kann, wobei die besagte Muffe (16) an jedem Ende eine Reihe von Transfixionsnadeln (22) aufweist, welche die überdeckenden Teile der Muffe fixieren, und welche regelmäßig kreisförmig und radial orientiert sind, **dadurch gekennzeichnet, dass** die Nadeln (22) ein blutstillendes Profil mit einem unteren Teil (32) mit kreisförmigem Querschnitt aufweisen, welcher übergeht in einen abschließend pyramidalförmigen Teil (34).

2. Vorrichtung gemäß Anspruch 1, **dadurch gekennzeichnet, dass** die erweiterbare Muffe (16) aus einem rautenförmig durchlöcherten Stahlzylinder (20) besteht, auf dem die Nadeln (22) an den Kanten der Rauten (20) aufgesetzt und durch schweißen oder kleben befestigt sind.

3. Vorrichtung gemäß Anspruch 1 oder 2, **dadurch gekennzeichnet, dass** die besagte Muffe (16) in einem Verhältnis des Durchmessers in fixierter Situation zum Durchmesser in ursprünglicher Situation von mehr als 2 erweiterbar ist.

4. Vorrichtung gemäß der Ansprüche 1 bis 3, **dadurch gekennzeichnet, dass** auch die Teile der Muffe (16), welche sich nicht an den Enden befinden, mit Transfixionsnadeln (22') versehen sind.

5. Vorrichtung gemäß Anspruch 4, **dadurch gekennzeichnet, dass** die Nadeln (22), welche kreisförmig an den Enden der Muffe (16) angebracht sind, geradlinig sind, und dass die anderen Transfixionsnadeln (22'), welche sich nicht an den Enden der Muffe (16) befinden, leicht geneigt sind, wobei ihre Spitzen zu dem einen oder dem anderen Ende der Muffe (16) oder in zufälliger Weise in alle möglichen anderen Richtungen zeigen.

6. Vorrichtung gemäß Anspruch 5, **dadurch gekennzeichnet, dass** die Transfixionsnadeln (22'), welche sich nicht an den Enden der Muffe (16) befinden, einen geneigten Endteil (34) mit einer Neigung zwischen 0 Grad und 10 Grad, vorzugsweise von 5 Grad, aufweisen.

7. Vorrichtung gemäß einem der Ansprüche 4 bis 6, **dadurch gekennzeichnet, dass** die Nadeln (22), welche sich an den Enden der Muffe (16) befinden, weniger hoch sind als die Transfixionsnadeln (22'), welche sich nicht an den Enden der Muffe (16) befinden.

8. Verbindungsvornchtung, verwendbar zur Ende-zu-Ende Anastomose von mindestens zwei Körperkanälen (12-1, 12-2, 12'-1, 12'-2, 12'-3) mittels einer Prothese (10,10'), deren Enden in die Endpartien der besagten Kanäle intubierbar sind, **dadurch gekennzeichnet, dass** die Vorrichtung aus einer Muffe (16) gemäß einem beliebigen der Ansprüche 1 bis 7 besteht, welche an jedem Ende der Prothese (10,10') in Hinblick auf die intubierten Teile einfügbar ist.
